# EUROPEAN PATENT APPLICATION

(11) **EP 4 450 075 A1**
(43) Date of publication of application: **23.10.2024**
(21) Application number: 22907466.1
(22) Date of filing: 14.12.2022
(51) Int. Cl.: A61K 35/28, A61P 1/16, A61P 9/10, C12N 5/077, C12N 5/0775

(54) **THERAPY FOR HEPATIC DISORDERS USING ADIPOSE-DERIVED MESENCHYMAL STEM CELL LINE**

(30) Priority: 16.12.2021 JP 2021204143
(71) Applicant: Keio University, Tokyo, 108-8345 (JP)
(72) Inventor: OBARA, Hideaki, Tokyo 160-8582 (JP); MATSUBARA, Kentaro, Tokyo 160-8582 (JP); FUKUDA, Kazumasa, Tokyo 160-8582 (JP); KITAGAWA, Yuko, Tokyo 160-8582 (JP); URUGA, Yukako, Tokyo 160-8582 (JP); MATSUBARA, Yumiko, Tokyo 160-8582 (JP)
(74) Representative: Hamer, Christopher K.
(86) International application number: PCT/JP2022/045961
(87) International publication number: WO 2023/112942

(57) **Abstract**

An object of the present invention is to provide, for example, a therapeutic agent for hepatic disorders using a specific mesenchymal stem cell line derived from an adipose tissue (ASCL), the therapeutic agent being capable of being preserved and stably supplied and having a better therapeutic effect on hepatic disorders. A feature of the present invention is to use a mesenchymal stem cell line derived from an adipose tissue, the mesenchymal stem cell line having been produced by a production method comprising: (A) inducing differentiation of one or more cells selected from a stromal vascular fraction of a vertebrate animal adipose tissue comprising a mesenchymal stem cell, an adipose progenitor cell, and a stromal cell into a mature adipocyte; and (B) inducing dedifferentiation of the mature adipocyte obtained in step (A) to obtain a mesenchymal cell line derived from the vertebrate animal adipose tissue.

## Description

### Technical Field

The present invention relates to, for example, a therapeutic agent for hepatic disorders, the therapeutic agent comprising a specific mesenchymal stem cell line derived from an adipose tissue as an active ingredient.

### Background Art

Hepatic disorders are caused by viral, alcoholic, nonalcoholic fatty, drug-induced, or autoimmune diseases or the like as well as induced by ischemia-reperfusion injury at the time of hepatectomy or liver transplantation. Highly useful treatment methods are still desired. In recent years, a method for treating hepatic disorders using mesenchymal stem cells (MSC) such as mesenchymal stem cells derived from an adipose tissue (also referred to as adipose-derived mesenchymal stem cells: ASC), which are proliferated by culture from an adipose tissue has also received global attention as a candidate thereof. For example, patent document 1 discloses a therapeutic agent and/or a prophylactic agent for liver diseases, comprising exosome derived from mesenchymal stem cells (MSC). However, a problem of this technique is that efficacy in treatment is not stable at a clinical use. Thus, the technique has not yet been established as a treatment method.

Meanwhile, the present inventors have previously developed a method for treating a wound by administering a platelet-like cell population produced from adipose tissue-derived mesenchymal stem cells to the wound (patent document 2). The present inventors have also developed a novel method for producing a mesenchymal stem cell line derived from an adipose tissue (adipose-derived mesenchymal stem cell line: ASCL) from vertebrate animal adipose tissue-derived mesenchymal stem cells or the like (i.e., a novel cell line establishing method) (patent document 3). The present inventors are pursuing clinical application as to a technique of producing platelet from ASCL.

### Prior Art Documents

### Patent Documents

Patent Document 1: Japanese unexamined Patent Application Publication No. 2021-116263
Patent Document 2: Japanese unexamined Patent Application Publication No. 2019-34917
Patent Document 3: Domestic re-publication No. 2017/094260 of PCT international application

### Summary of the invention

### Object to be Solved by the Invention

A treatment method using mesenchymal stem cells derived from an adipose tissue (ASC) has a strong tendency for the therapeutic effect of ASC to depend on the quality of donor cells, and presents problems such as the resulting therapeutic effect largely varying in degree and difficult stable supply of ASC.

An object of the present invention is to provide, for example, a therapeutic agent for hepatic disorders using a specific mesenchymal stem cell line derived from an adipose tissue (ASCL), the therapeutic agent being capable of being preserved and stably supplied and having a better therapeutic effect on hepatic disorders.

### Means to Solve the Object

The present inventors conducted extensive studies to solve the above problems and found that, for example, use of ASCL compared with use of ASC exerts a better therapeutic effect in the treatment of hepatic disorders such as liver ischemia-reperfusion injury, whereby the present invention was accomplished. ASCL is a cell line and is capable of being more stably supplied as compared with ASC.

More specifically, the present invention relates to:
(1) a therapeutic agent for hepatic disorders, comprising a mesenchymal stem cell line derived from an adipose tissue as an active ingredient, the mesenchymal stem cell line having been produced by a production method comprising the following steps (A) and (B):
   (A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
   (B) a step of inducing differentiation of the mature adipocyte obtained in step (A) to obtain a mesenchymal cell line derived from the vertebrate animal adipose tissue;
(2) the therapeutic agent for hepatic disorders according to the above (1), wherein the step of inducing differentiation of one or more cells into a mature adipocyte in step (A) is a step of culturing the one or more cells in basal medium for mesenchymal cell culture comprising one or more adipocytes differentiation inducing substances selected from the group consisting of dexamethasone, isobutylmethylxanthine, insulin, and serum;
(3) the therapeutic agent for hepatic disorders according to the above (1) or (2), wherein the dedifferentiation induction of the mature adipocyte in step (B) is performing ceiling culture of the mature adipocyte;
(4) the therapeutic agent for hepatic disorders according to any one of the above (1) to (3), wherein the one or more cells are cells obtained by removing the mature adipocyte from a cell population obtained by treating the vertebrate animal adipose tissue with an enzyme capable of dispersing the vertebrate animal adipose tissue cells;
(5) the therapeutic agent for hepatic disorders according to any one of the above (1) to (4), wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain;
(6) the therapeutic agent for hepatic disorders according to any one of the above (1) to (5), wherein the mesenchymal stem cell line derived from an adipose tissue expresses one or more surface markers selected from the following surface marker group of mesenchymal cells, and does not express one or more surface markers selected from the following surface marker group of blood cells:
   surface marker group of mesenchymal cells: CD13, CD29, CD44, CD71, CD73, CD90, CD105, CD166, HLA-ABC;
   surface marker group of blood cells: CD11b, CD14, CD19, CD34, CD41, CD42b, CD45, CD56, HLA-DR;
(7) the therapeutic agent for hepatic disorders according to any one of the above (1) to (6), wherein the hepatic disorders are hepatic ischemia-reperfusion injury; and
(8) the therapeutic agent for hepatic disorders according to any one of the above (1) to (7), wherein the vertebrate animal is a human.

### Effect of the Invention

The present invention can provide, for example, a therapeutic agent for hepatic disorders using a specific mesenchymal stem cell line derived from an adipose tissue (ASCL), the therapeutic agent being capable of being preserved and stably supplied and having a better therapeutic effect on hepatic disorders.

### Brief Description of Drawings

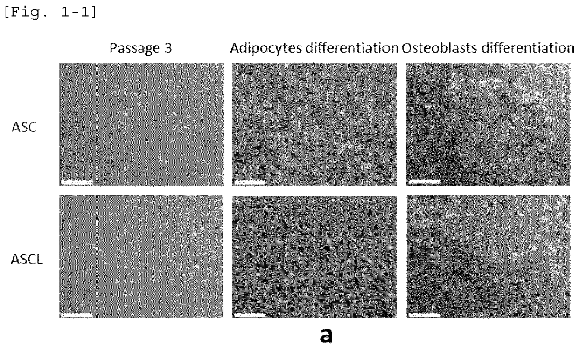

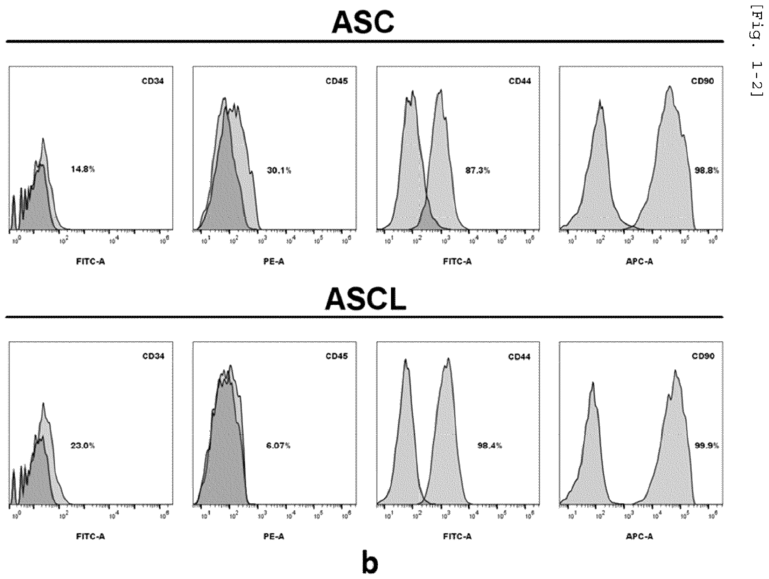

[Figure 1-1] - [Figure 1-2] Figure 1 illustrates results about the characterization of rat ASC and ASCL. (a) Passage-3 rat ASC and ASCL were induced to differentiate into adipocytes and osteoblasts. The cells were stained with Oil Red stain and alkaline phosphatase, respectively. Scale bar = 100 um. (b) Cell surface antigens of rat ASC and ASCL were analyzed. Passage-3 cells were collected, and surface antigens were reacted with specific antibodies labeled with a fluorescent dye, and analyzed by flow cytometry. For CD34, CD44, and CD90 in ASC and CD34, CD44, and CD90 in ASCL, the relatively left peak depicts a negative control surface marker population, and the relatively right peak depicts each surface marker population expressed in ASC or ASCL cells. For CD45 in ASC and ASCL, the relatively left peak depicts each surface marker population expressed in ASC or ASCL cells, and the relatively right peak depicts a negative control surface marker population. The number in each panel represents mean fluorescence intensity of the marker expressed in ASC or ASCL.

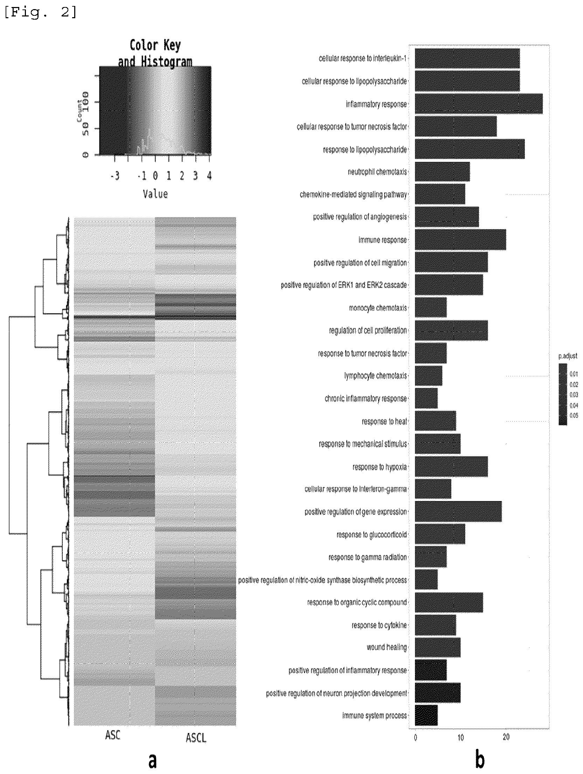

[Figure 2] Figure 2 illustrates results about the RNA-seq (transcriptome analysis) of rat ASC and ASCL. RNA for sequence analysis was extracted from passage-3 rat ASC and ASCL. (a) Genes differentially expressed between ASCL and ASC are illustrated. (b) Results about top 30 based on the lowest false discovery rate (FDR) in the gene ontology (GO) analysis of ASCL and ASC are illustrated. Each OG term of Figure 2b means cellular response to interleukin-1, cellular response to lipopolysaccharide, inflammatory response, cellular response to tumor necrosis factor, response to lipopolysaccharide, neutrophil chemotaxis, chemokine-mediated signaling pathway, positive regulation of angiogenesis, immune response, positive regulation of cell migration, positive regulation of ERK1 and ERK2 cascade, monocyte chemotaxis, regulation of cell proliferation, response to tumor necrosis factor, lymphocyte chemotaxis, chronic inflammatory response, response to heat, response to mechanical stimulus, response to hypoxia, cellular response to interferon-γ, positive regulation of gene expression, response to glucocorticoid, response to gamma radiation, positive regulation of nitric oxide synthase biosynthetic process, response to organic cyclic compound, response to cytokine, wound healing, positive regulation of inflammatory response, positive regulation of neuron projection development, and immune system process in order from the top.

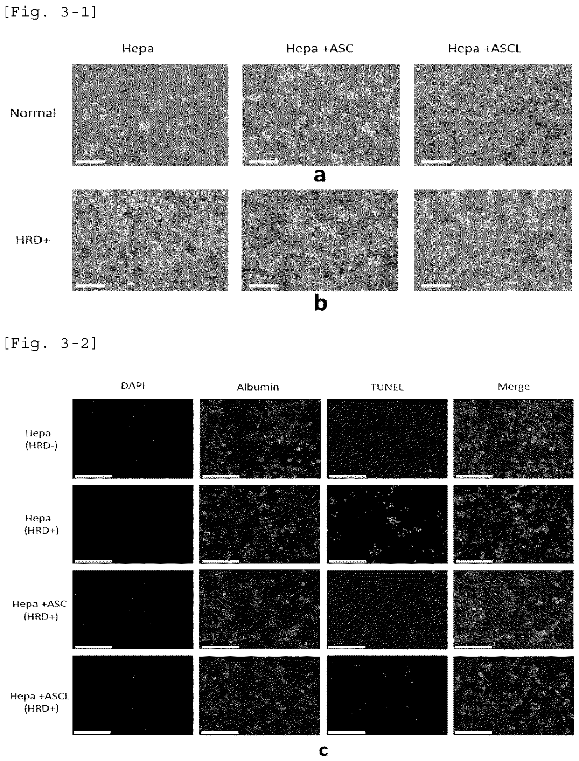

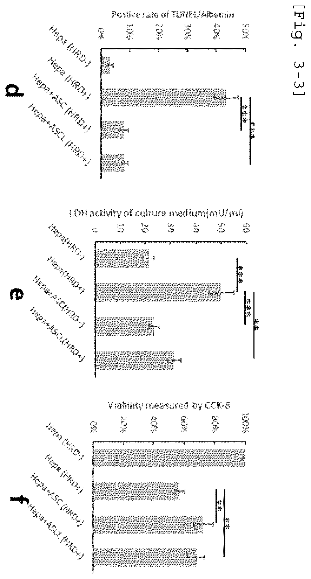

[Figure 3-1] - [Figure 3-3] Figure 3 is a diagram showing that the coculture of ASC or ASCL with hepatocytes can relieve hypoxia/reoxygenation damage in the hepatocytes. In Figures 3(d) to 3(f), each data represents mean ± SD. *p < 0.05, **p < 0.01, ***p < 0.001. (a) Micrographs taken when rat hepatocytes were cocultured with rat ASC or ASCL are shown (the monoculture of rat hepatocytes was used as a control group) . Bar = 100 um. "Hepa" represents the monoculture of hepatocytes, "Hepa+ASC" represents the coculture of hepatocytes and ASC, and "Hepa+ASCL" represents the coculture of hepatocytes and ASCL. (b) Micrographic images of monoculture groups and coculture groups after induction of hypoxia for 3 hours and reoxygenation damage for 1 hour (HRD) are shown. Bar = 100 um. "Hepa" represents the monoculture of hepatocytes, "Hepa+ASC" represents the coculture of hepatocytes and ASC, and "Hepa+ASCL" represents the coculture of hepatocytes and ASCL. (c) The diagram illustrates results of staining monoculture groups and coculture groups with albumin (Cy5), TUNEL (GFP), and DAPI. Bar = 125 um. "Hepa(HRD-)" represents the monoculture of hepatocytes having no HRD, "Hepa(HRD+)" represents the monoculture of hepatocytes having HRD, "Hepa+ASC(HRD+)" represents the coculture of hepatocytes having HRD and ASC, and "Hepa+ASCL(HRD+)" represents the coculture of hepatocytes having HRD and ASCL. (d) A positive rate of DNA fragmentation in hepatocytes was calculated from the ratio of GFP-positive cells to albumin-positive cells (Cy5) by use of a TUNEL method. (e) LDH activity in each medium of a culture group after HRD is shown (n = 3). (f) Viability measured by CCK-8 is shown.

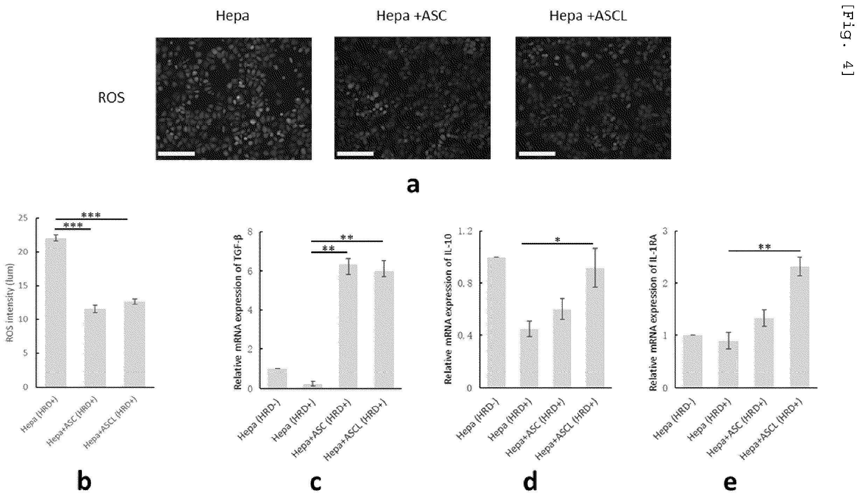

[Figure 4] Figure 4 is a diagram showing that ASCL and ASC protest hepatocytes from reactive oxygen species damage through the immune regulation of cytoprotective cytokines, and alleviate damage of hypoxic reactivation. (a) and (b) ROS strength of each group after HRD is illustrated. (c) Relative mRNA expression of TGF-β was detected by qRT-PCR and compared with a group without HRD. The results are shown. (d) Relative mRNA expression of IL-10 was detected by qRT-PCR and compared with a group without HRD. The results are shown. (e) Relative mRNA expression of IL-1Ra was detected by qRT-PCR and compared with a group without HRD. The results are shown. Each data of (b) to (e) represents mean ± SD. *p < 0.05, **p < 0.01, ***p < 0.001.

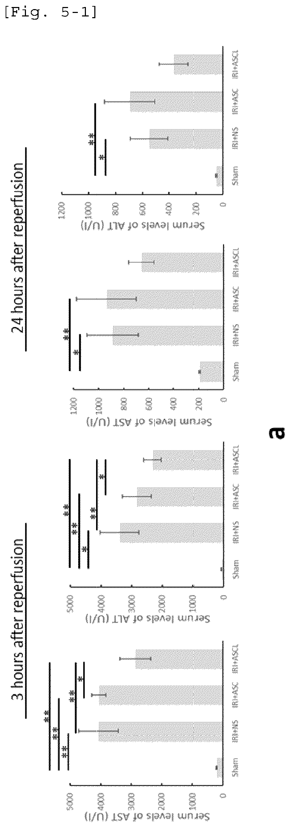

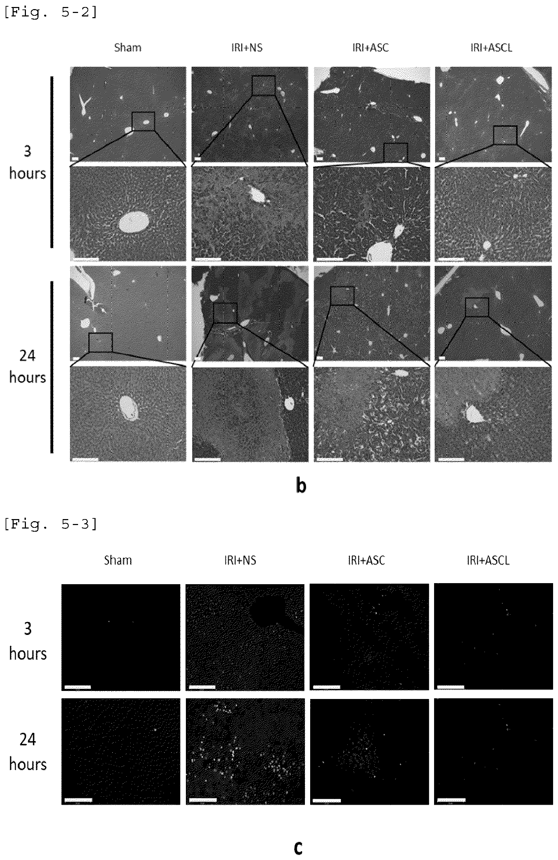

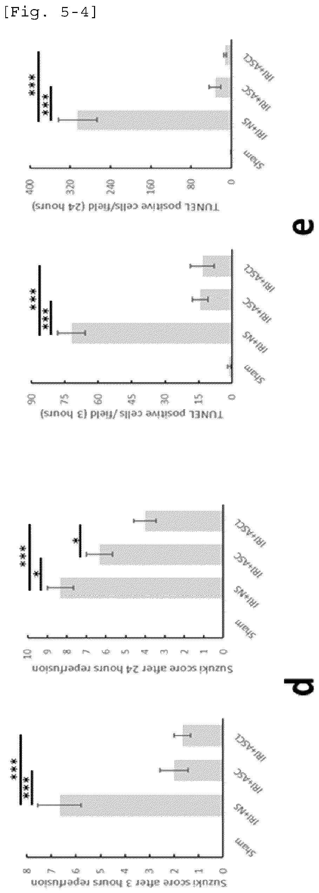

[Figure 5-1] - [Figure 5-4] Figure 5 is a diagram illustrating a protective effect of ASCL on hepatic ischemia-reperfusion injury (IRI) *in vivo.* (a) Serum levels of AST and ALT in each group are illustrated which were measured 3 hours and 24 hours after hepatic ischemia-reperfusion injury. (b) Results of performing H.E. staining of hepatic tissues in each group 3 hours and 24 hours after hepatic ischemia-reperfusion injury are illustrated. Bar = 125 um. (c) Results of performing TUNEL staining of damaged hepatic tissues 3 hours and 24 hours after hepatic ischemia-reperfusion injury are illustrated. Bar = 125 um. (d) Results of analyzing change in sinusoidal capillarization and hepatocyte vacuolization and necrosis in accordance with Suzuki score are illustrated. (e) Results of calculating the cell death of hepatic tissues in terms of the number of GFP-positive cells per field by a TUNEL method are illustrated. In (d) and (e), data represents a mean ± SD. *p < 0.05, **p < 0.01, ***p < 0.001.

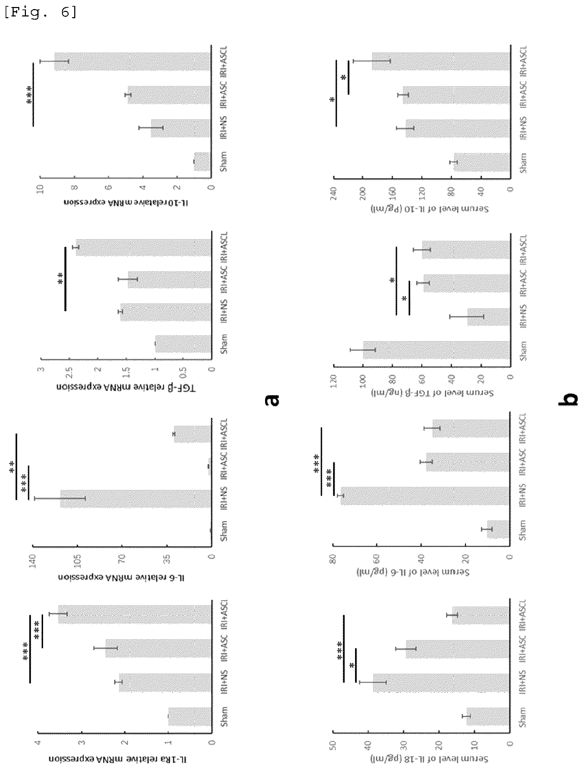

[Figure 6] Figure 6 is a diagram illustrating a prophylactic effect of ASCL on hepatic ischemia-reperfusion injury in *vivo.* (a) Results of detecting relative mRNA expression of IL-1Ra, IL-6, TGF-β, and IL-10 in each lobule that underwent hepatic ischemia-reperfusion injury by quantitative PCR are illustrated. (b) Results of measuring serum levels of IL-18, IL-6, TGF-β, and IL-10 by ELISA are illustrated. In (a) and (b), data represents a mean ± SD. *p < 0.05, **p < 0.01, ***p < 0.001.

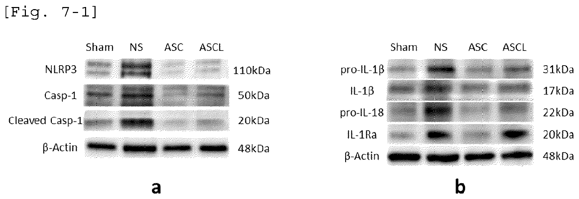

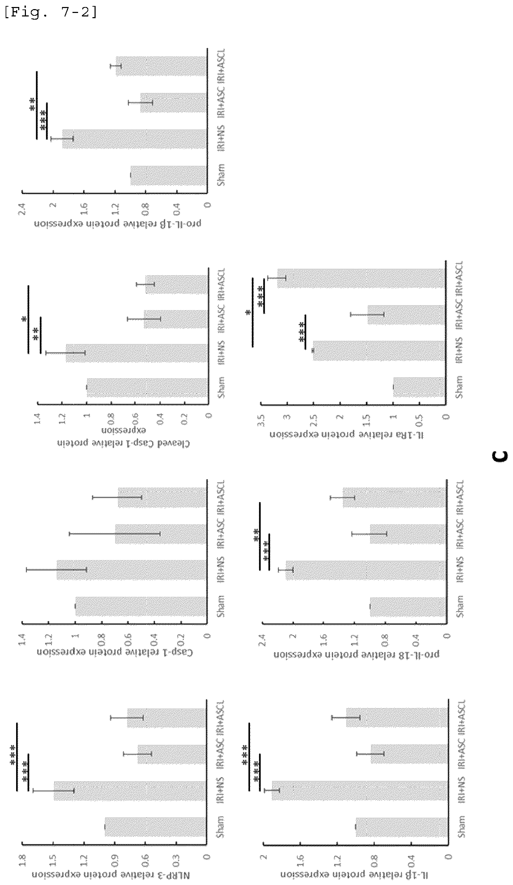

[Figure 7-1] - [Figure 7-2] Figure 7 is a diagram showing that pretreatment of ASCL inhibits NLRP3 inflammasome and activates the expression of IL-1RA. (a) and (b) Relative protein expression of NLRP-3-related proteins was detected by Western blot analysis from hepatic tissues of each group after hepatic ischemia-reperfusion injury. The results are shown. (c) Mean signal intensity of bands in Western blotting was quantified using β-actin as an internal standard. The results are shown. Data represents mean ± SD. *p < 0.05, **p < 0.01, ***p < 0.001.

### Mode of Carrying Out the Invention

The present invention includes an embodiment of
[1] a therapeutic agent for hepatic disorders (hereinafter also referred to as "therapeutic agent of the present invention" in the present specification), the therapeutic agent comprising a mesenchymal stem cell line derived from an adipose tissue as an active ingredient, the mesenchymal stem cell line having been produced by a production method comprising the following steps (A) and (B):
   (A) inducing differentiation of one or more cells selected from a stromal vascular fraction of a vertebrate animal adipose tissue comprising a mesenchymal stem cell, an adipose progenitor cell, and a stromal cell into a mature adipocyte; and
   (B) inducing dedifferentiation of the mature adipocyte obtained in step (A) to obtain a mesenchymal cell line derived from the vertebrate animal adipose tissue.

The present invention also includes, as other embodiments, embodiments of:
[2] a method for treating hepatic disorders, the method comprising the step of administering a mesenchymal stem cell line derived from an adipose tissue to a subject in need of treatment of the hepatic disorders, the mesenchymal stem cell line having been produced by a production method comprising the above steps (A) and (B);
[3] a mesenchymal stem cell line derived from an adipose tissue for use in treatment of hepatic disorders, wherein the mesenchymal stem cell line derived from an adipose tissue has been produced by a production method comprising the above steps (A) and (B);
[4] use of a mesenchymal stem cell line derived from an adipose tissue for producing a therapeutic agent for hepatic disorders, wherein the mesenchymal stem cell line derived from an adipose tissue has been produced by a production method comprising the above steps (A) and (B);
[5] a mesenchymal stem cell line derived from an adipose tissue for use as a therapeutic agent for hepatic disorders, wherein the mesenchymal stem cell line derived from an adipose tissue has been produced by a production method comprising the above steps (A) and (B); etc.

The therapeutic agent of the present invention is not particularly limited as long as the therapeutic agent is a therapeutic agent for hepatic disorders, the therapeutic agent comprising a mesenchymal stem cell line derived from an adipose tissue as an active ingredient, the mesenchymal stem cell line having been produced by a production method comprising the following steps (A) and (B):
(A) inducing differentiation of one or more cells selected from a stromal vascular fraction of a vertebrate animal adipose tissue comprising a mesenchymal stem cell, an adipose progenitor cell, and a stromal cell into a mature adipocyte; and
(B) inducing dedifferentiation of the mature adipocyte obtained in step (A) to obtain a mesenchymal cell line derived from the vertebrate animal adipose tissue.

The mature adipocyte obtained by the differentiation induction in step (A) is a mature adipocyte more easily dedifferentiated (hereinafter also referred to as "easy-to-dedifferentiate mature adipocyte" in the present Description) than a mature adipocyte which have been present in a vertebrate animal adipose tissue, and thus the dedifferentiation induction in step (B) presumably enables the produce (establishment) of a mesenchymal cell line derived from a vertebrate animal adipose tissue (ASCL) more simply, in a shorter period of time, and more efficiently. The present inventors have already confirmed that ASCL produced from the easy-to-dedifferentiate mature adipocyte (i.e., ASCL having been produced by the production method according to the present invention) differs as a cell from a mesenchymal stem cell line derived from an adipose tissue produced by direct dedifferentiation induction of a mature adipocyte obtained from a vertebrate animal adipose tissue. The "ASCL" as used herein refers to a mesenchymal stem cell line derived from an adipose tissue having been produced by the production method according to the present invention.

The above production method according to the present invention can be an ex vivo or in vitro production method.

### (Method for producing cell line according to present invention)

The mesenchymal stem cell line derived from an adipose tissue according to the present invention is a cell line having been produced by the method for producing a cell line according to the present invention. The method for producing a cell line according to the present invention is not particularly limited as long as it is a production method comprising the above steps (A) and (B).

### (Step A)

The above step (A) is not particularly limited as long as it is a step of inducing differentiation of one or more cells selected from a stromal vascular fraction of a vertebrate animal adipose tissue comprising a mesenchymal stem cell, an adipose progenitor cell, and a stromal cell (also referred to as "mesenchymal stem cells" in the present Description) into a mature adipocyte. The step of differentiation induction is an ex vivo or in vitro step of differentiation induction.

The species from which the adipose tissue is derived is not particularly limited as long as a species is a vertebrate animal, and examples include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable. For a mesenchymal stem cell line derived from a vertebrate animal adipose tissue produced by the method for producing a cell line of the present invention, it is preferable to use the adipose tissue of the target vertebrate animal in the method for producing a cell line of the present invention in light of minimizing a rejection reaction or the like.

The "adipose tissue" as used herein is not particularly limited as long as a tissue comprises fats and examples include a subcutaneous adipose tissue, an adipose tissue in the bone marrow, and a visceral adipose tissue, with a subcutaneous adipose tissue being preferable in light of comparatively low invasiveness to a vertebrate animal supplying the adipose tissue and being comparatively easily collectable.

The "stromal vascular fraction" as used herein means the cells other than mature adipocytes among the cells of a vertebrate animal adipose tissue. A stromal vascular fraction typically comprises cells such as a mesenchymal stem cell, an adipose progenitor cell, a stromal cell, a vascular endothelial cell, a cell related to blood, a smooth muscle cell, and a fibroblast. The "stromal vascular fraction" can be obtained by removing mature adipocytes from a cell population obtained by treating a vertebrate animal adipose tissue with an enzyme capable of dispersing the vertebrate animal adipose tissue cells.

The above "one or more cells selected from a stromal vascular fraction of a vertebrate animal adipose tissue comprising a mesenchymal stem cell, an adipose progenitor cell, and a stromal cell" are not limited as long as one or more cells are selected from a stromal vascular fraction of a vertebrate animal adipose tissue comprising a mesenchymal stem cell, a preadipocyte or an adipose progenitor cell, and a stromal cell, but in light of more efficiently producing a mesenchymal cell line derived from a vertebrate animal adipose tissue, the cell population, rather than comprising only an adipose progenitor cell, preferably comprises at least an adipose progenitor cell and a mesenchymal stem cell and/or a stromal cell, with the cell population comprising at least an adipose progenitor cell, a mesenchymal stem cell, and a stromal cell being more preferable, with the cell population of a stromal vascular fraction being further preferable in light of easy preparation.

Further, examples of the preferable embodiment of the above "one or more cells selected from a stromal vascular fraction of a vertebrate animal adipose tissue comprising a mesenchymal stem cell, an adipose progenitor cell, and a stromal cell" include one or more cells selected from a stromal vascular fraction comprising a mesenchymal stem cell, an adipose progenitor cell, and a stromal cell obtained by dispersing cells of the vertebrate animal adipose tissue, of which a cell population obtained by removing mature adipocytes from a cell population obtained by treating the vertebrate animal adipose tissue with an enzyme capable of dispersing the vertebrate animal adipose tissue cells (cell population A) being preferable. A cell population obtained by further removing vascular endothelial cells and/or cells related to blood from the cell population A may also be used. The above cell population (cell population A) obtained by removing mature adipocytes from a cell population obtained by treating a vertebrate animal adipose tissue with an enzyme capable of dispersing the vertebrate animal adipose tissue cells is a cell population of stromal vascular fractions, which typically comprises cells such as a mesenchymal stem cell, an adipose progenitor cell, a stromal cell, a vascular endothelial cell, a cell related to blood, a smooth muscle cell and a fibroblast of a vertebrate animal adipose tissue.

Examples of the above "treating a vertebrate animal adipose tissue with an enzyme capable of dispersing vertebrate animal adipose tissue cells" include a method in which a vertebrate animal adipose tissue is immersed in a solution comprising such an enzyme and incubated, for example, for about 30 minutes to 3 hours.

The above "enzyme capable of dispersing vertebrate animal adipose tissue cells" is not particularly limited as long as it can disperse cells of a vertebrate animal adipose tissue when allowed to act on the vertebrate animal adipose tissue, and examples include one or more enzymes selected from the group consisting of collagenase, trypsin, caseinase, clostripain, trypsin-EDTA, dispase, thermolysin, pronase, hyaluronidase, pancreatin, elastase, and papain, of which at least one or more enzymes selected from the group consisting of collagenase, trypsin, caseinase, and clostripain being preferable, and commercial collagenase (type I) and collagenase (type II) being more preferable, with collagenase (type II) being further preferable. Further, the above "enzyme capable of dispersing vertebrate animal adipose tissue cells" preferably comprises at least collagenase.

The above "removing mature adipocytes from a cell population obtained by treating a vertebrate animal adipose tissue with an enzyme capable of dispersing the vertebrate animal adipose tissue cells" is not particularly limited as long as a method can remove mature adipocytes from such a cell population, but examples preferably include a method of recovering a cell population (cell pellet) which is precipitated by centrifugation of a suspension comprising the above cell population. Mature adipocytes comprise a large amount of fats, thus have a light specific gravity and float in the upper part of supernatant when centrifuged, hence the recovery of a cell pellet precipitated by the centrifugation enables the removal of mature adipocytes. Further, the method for removing vascular endothelial cells, smooth muscle cells, and fibroblasts from the cell population obtained by treating a vertebrate animal adipose tissue with an enzyme capable of dispersing vertebrate animal adipose tissue cells is not particularly limited as long as a method can remove these cells from such a cell population and examples include a method for removing vascular endothelial cells from the cell population when CD31 known as a surface marker of the vascular endothelial cell selects negative cells (or CD31 removes positive cells), and examples of the method for removing cells related to blood include a method for removing cells related to blood from the cell population by selecting CD45- (a surface marker of hematopoietic cells other than red blood cell and platelet) negative and Ter119- (a surface marker of red blood cell and progenitor cell thereof) negative cells (or CD45-positive and Ter119-positive cells are removed). Additionally, when 7-amino-actinomycin D (7-AAD), which is not a surface marker, being negative is used as an indicator, it is preferable because dead cells comprised in a vertebrate animal adipose tissue can be excluded. 7-AAD intercalates with a DNA chain of a dead cell and produces red fluorescence at a 488-nm excitation light.

The above precipitated cell pellet (cell population A) is cells of a stromal vascular fraction, which typically comprises a mesenchymal stem cell, an adipose progenitor cell, a stromal cell (a stroma cell), a vascular endothelial cell, a smooth muscle cell, and a fibroblast, however, those of which differentiable into a mature adipocyte is a mesenchymal stem cell, an adipose progenitor cell, and a stromal cell. For this reason, a further step may or may not be included for removing any one or more, or all kinds, of the cells other than these 3 kinds from the above precipitated cell pellet before the differentiation induction into a mature adipocyte is carried out, but it is preferable not to include such a step in light of convenience of operation. A vascular endothelial cell, a smooth muscle cell, and a fibroblast do not differentiate into a mature adipocyte even when induced with mesenchymal stem cells to differentiate into a mature adipocyte or do not interfere in the differentiation of mesenchymal stem cells into mature adipocytes.

In the above step (A), examples of the method for inducing differentiation of one or more cells selected from a stromal vascular fraction of a vertebrate animal adipose tissue comprising a mesenchymal stem cell, an adipose progenitor cell, and a stromal cell into a mature adipocyte preferably include a method for culturing the one or more cells selected from a stromal vascular fraction of a vertebrate animal adipose tissue comprising a mesenchymal stem cell, an adipose progenitor cell, and a stromal cell in basal medium for mesenchymal cell culture comprising adipose cell differentiation inducing substances. The method for culturing mesenchymal stem cells in basal medium for mesenchymal cell culture comprising adipose cell differentiation inducing substances is not particularly limited as long as a method can induce differentiation of mesenchymal cells into mature adipocytes by such a culture, and the same method as the typical method for inducing differentiation of an adipose progenitor cell into a mature adipocyte, that is, a method for culturing a starting cell in basal medium for mesenchymal cell culture comprising adipose cell differentiation inducing substances can be used.

In the above step (A), examples of the conditions for culturing mesenchymal stem cells in basal medium for mesenchymal cell culture comprising adipose cell differentiation inducing substances include a method of adhesion culture in a culture vessel coated with the extracellular matrix, and examples of the culture temperature include typically a range from 12 to 45°C, preferably a range from 15 to 37°C, and examples of the culture period include, in light of the balance between producing a mesenchymal cell line derived from a vertebrate animal adipose tissue more efficiently and producing in a shorter period of time, a range from 5 to 16 days, preferably a range from 7 to 14 days, more preferably a range from 8 to 12 days, further preferably a range from 9 to 11, more preferably for 10 days. In the culture, mesenchymal stem cells may or may not be subcultured. Further, examples of the above extracellular matrix include at least one or more components selected from collagen, fibronectin, proteoglycan, and laminin, and BD Matrigel (registered trademark) (manufactured by BD Biosciences) comprising these components can also be used.

The above adipose cell differentiation inducing substances are not particularly limited as long as a substance has an action to differentiate a cell inducible to differentiate into a mature adipocyte or has an assisting action on the action, and examples include one or more substances selected from the group consisting of dexamethasone, isobutylmethylxanthine, insulin, and serum, of which, in light of obtaining a good differentiation inducing efficiency into a mature adipocyte, "combination of serum and dexamethasone", "combinations of adipose cell differentiation inducing substances comprising at least serum and dexamethasone", "combination of serum and isobutylmethylxanthine", and "combinations of adipose cell differentiation inducing substances comprising at least serum and isobutylmethylxanthine" being preferable, of which "combination of serum, dexamethasone, and insulin", "combinations of adipose cell differentiation inducing substances comprising at least serum, dexamethasone, and insulin", "combination of serum, isobutylmethylxanthine, and insulin", "combinations of adipose cell differentiation inducing substances comprising at least serum, isobutylmethylxanthine and insulin", "combination of serum, dexamethasone, and isobutylmethylxanthine", and "combinations of adipose cell differentiation inducing substances comprising at least serum, dexamethasone, and isobutylmethylxanthine" being more preferable, of which "combination of serum, dexamethasone, isobutylmethylxanthine, and insulin", "combinations of adipose cell differentiation inducing substances comprising at least serum, dexamethasone, isobutylmethylxanthine, and insulin" being further preferable. The adipose cell differentiation inducing substances and the basal medium for mesenchymal cell culture comprising such a substance may be commercial products, or a medium prepared by adding an adipose cell differentiation inducing substance to the basal medium for mesenchymal cell culture may be used as such a medium. Examples of the commercial medium comprising an adipose cell differentiation inducing substance preferably include Adipocyte Differentiation Medium (manufactured by Cell Applications, Inc.). Examples of the substance having an assisting action on the action to differentiate into a mature adipocyte other than the above listed adipose cell differentiation inducing substances include Rosiglitazone, Pioglitazone, and Indomethacin.

The concentration of the above adipose cell differentiation inducing substances in the medium is not particularly limited as long as a concentration can induce mesenchymal stem cells into mature adipocytes but examples include, in terms of dexamethasone concentration, typically a range from 0.1 to 10 µM, preferably a range from 0.5 to 2.5 µM, in terms of isobutylmethylxanthine concentration, a range from 10 to 1000 µM, preferably a range from 250 to 750 µM, in terms of insulin concentration, a range from 0.1 to 10 µM, preferably a range from 0.5 to 2.5 µM, and in terms of serum concentration, a range from 1 to 20 wt%, preferably a range from 5 to 15 wt%, more preferably 7 to 13 wt%.

The "basal medium for mesenchymal cell culture" in the present Description is not particularly limited as long as medium can culture at least 1 kind of the mesenchymal cells therein and proliferate the mesenchymal cell, but in light of easy preparation and preventing lot-to-lot variation, a chemically synthesized medium is preferable, and the medium preferably comprises one or more saccharide(s), one or more inorganic salt(s), one or more amino acid(s), and one or more vitamin(s), and one or more other components as needed.

Examples of the above saccharide specifically include a monosaccharide such as glucose, mannose, fructose and galactose, and a disaccharide such as sucrose, maltose, and lactose, of which glucose being particularly preferable, and one or more of these saccharides can be added in combination.

Examples of the above inorganic salt specifically include one or more inorganic salt(s) such as calcium chloride, calcium nitrate, a copper sulfate pentahydrate, an iron(III) nitrate nonahydrate, an iron (II) sulfate heptahydrate, a magnesium chloride hexahydrate, magnesium sulfate, potassium chloride, sodium chloride, sodium bicarbonate, disodium hydrogen phosphate, a disodium hydrogenphosphate dihydrate, sodium dihydrogen phosphate, a sodium dihydrogen phosphate monohydrate, a sodium dihydrogen phosphate dihydrate, a sodium selenite pentahydrate, and a zinc sulfate heptahydrate.

Examples of the above amino acids specifically include one or more amino acid(s) selected from alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and preferably include an L-form amino acid and a derivative thereof and a by-product such as a salt thereof and a hydrate thereof. Examples of the above arginine include an arginine by-product such as an L-arginine hydrochloride and an L-arginine monohydrochloride, examples of the above aspartic acid include an aspartic acid by-product such as an L-sodium aspartate monohydrate, an L-aspartic acid monohydrate, potassium L-aspartate, and magnesium L-aspartate, examples of the above cysteine include a cysteine by-product such as L-cysteine dihydrochloride and an L-cysteine hydrochloride monohydrate, and a lysine by-product such as L-lysine hydrochloride, examples of the above glutamic acid include a glutamine by-product such as monosodium L-glutamate, examples of the above asparagine include an asparagine by-product such as an L-asparagine monohydrate, examples of the above tyrosine include a tyrosine by-product such as an L-tyrosine disodium dihydrate, examples of the above histidine include a histidine by-product such as histidine hydrochloride and a histidine hydrochloride monohydrate, and examples of the above lysine include a lysine by-product such as L-lysine hydrochloride.

Examples of the above vitamins specifically include one or more vitamin(s) selected from biotin, choline, folic acid, inositol, niacin, pantothenic acid, pyridoxine, riboflavin, thiamine, vitamin B12, paraaminobenzoic acid (PABA), and ascorbic acid, and a derivative each thereof and a by-product thereof such as a salt thereof and a hydrate thereof. Examples of the above choline include a choline by-product such as choline chloride, examples of the niacin include a niacin by-product such as nicotinic acid, nicotinamide, and nicotinic alcohol, examples of the pantothenic acid include a pantothenic acid by-product such as calcium pantothenate, sodium pantothenate, and panthenol, examples of the pyridoxine include a pyridoxine by-product such as pyridoxine hydrochloride, pyridoxal hydrochloride, pyridoxal phosphate, and pyridoxamine, examples of the thiamine include a thiamine by-product such as thiamine hydrochloride, thiamine nitrate, bisthiamine nitrate, a thiamine dicetyl sulfate ester salt, fursultiamine hydrochloride, octothiamine, and benfotiamine, examples of the ascorbic acid include an ascorbic acid by-product such as ascorbic acid 2-phosphate, ascorbic acid magnesium phosphate, sodium ascorbate sulfate, aminopropyl ascorbyl phosphate, and sodium ascorbate phosphate.

Examples of the above other components include a buffer such as HEPES, an antibiotic such as penicillin and streptomycin, pyruvic acid and a derivative thereof and a by-product thereof such as a salt thereof and a hydrate thereof, and phenol red, examples of the above antibiotics include penicillin G sodium and streptomycin sulfate, or preferably a penicillin-streptomycin solution, and examples of the pyruvic acid by-product preferably include sodium pyruvate.

Specific examples of the above basal medium for mesenchymal cell culture include a known commercial chemically synthesized medium such as Dulbecco's modified Eagle's medium (DMEM), Iscove's Modified Dulbecco's Medium (IMDM), RPMI1640 medium, minimum essential medium (MEM), basal medium of Eagle (BME), and F12 medium, a medium of 2 or more of these medium mixed in a suitable ratio such as DMEM/F12 (medium of DMEM and F12 medium mixed in 1:1), with medium to which one or more substances selected from the group consisting of antibiotics such as penicillin and streptomycin; and additional amino acids (preferably non-essential amino acids); are added being preferable, and medium wherein an antibiotic (preferably penicillin G sodium, streptomycin sulfate or a penicillin-streptomycin solution) is further added to DMEM, IMDM or RPMI1640 medium being particularly more preferable, of which medium wherein an antibiotic (preferably penicillin G sodium, streptomycin sulfate, or a penicillin-streptomycin solution) is further added to DMEM being particularly preferable.

Examples of the particularly preferable basal medium for mesenchymal cell culture in the present invention include medium wherein 100 U/mL (final concentration) of a penicillin-streptomycin solution is added to DMEM having the composition to be described later (hereinafter referred to as "particularly preferable basal medium in the present invention"), and medium comprising each component in a concentration of the proportion ranging independently from 70% to 130% to the concentration of each component in the particularly preferable basal medium in the present invention.

### (Composition of DMEM)

200 mg/L of anhydrous calcium chloride, 0.1 mg/L of Fe(NO₃)₃•9H₂O, 200 mg/L of potassium chloride, 97.67 mg/L of anhydrous magnesium sulfate, 6400 mg/L of sodium chloride, 3700 mg/L of sodium bicarbonate, 125 mg/L of sodium dihydrogen phosphate monohydrate, 4500 mg/L of D-glucose, 15 mg/L of phenol red, 110 mg/L of sodium pyruvate, 84 mg/L of L-arginine hydrochloride, 63 mg/L of L-cysteine dihydrochloride, 584 mg/L of L-glutamine, 30 mg/L of glycine, 42 mg/L of L-histidine hydrochloride monohydrate, 105 mg/L of L-isoleucine, 105 mg/L of L-leucine, 146 mg/L of L-lysine hydrochloride, 30 mg/L of L-methionine, 66 mg/L of L-phenylalanine, 42 mg/L of L-serine, 95 mg/L of L-threonine, 16 mg/L of L-tryptophan, 104 mg/L of L-tyrosine disodium dihydrate, 94 mg/L of L-valine, 4 mg/L of D-calcium pantothenate, 4 mg/L of choline chloride, 4 mg/L of folic acid, 7.2 mg/L of i-inositol, 4 mg/L of nicotinamide, 4 mg/L of pyridoxine hydrochloride, 0.4 mg/L of rivoflavin, 4 mg/L of thiamine hydrochloride.

Mature adipocytes obtained in the above step (A) (that is, the mature adipocyte population comprising mature adipocytes) are easy-to-dedifferentiate mature adipocytes, which are comparatively easily dedifferentiated when induced to dedifferentiate (that is, easy-to-dedifferentiate mature adipocyte population comprising easy-to-dedifferentiate mature adipocytes). The "easy-to-dedifferentiate mature adipocyte population" in the present Description means a mature adipocyte population having a proportion of the cell line obtained which is 1.5 or more times more than that of the mature adipocyte population collected from a vertebrate animal adipose tissue as described in the conventional method (Japanese Patent No. 5055611), and includes mature adipocyte populations having preferably 2 or more times, more preferably 4 or more times, further preferably 6 or more times, more preferably 10 or more times, and further preferably 15 or more times proportions. The above "proportion of the cell line obtained" indicates the proportion of cell line obtained from a specific amount of a mature adipocyte population, and the proportion preferably includes, for example, a proportion (rate) of "a weight of a cell line to be obtained" to "a weight of mature adipocytes to be used for dedifferentiation induction."

### (Step B)

The above step (B) is not particularly limited as long as a step can induce dedifferentiation of the mature adipocytes (easy-to-dedifferentiate mature adipocytes) obtained in step (A) to obtain a mesenchymal cell line derived from a vertebrate animal adipose tissue. The step is an ex vivo or in vitro step.

Mature adipocytes used in step (B) is the mature adipocytes obtained by the differentiation induction in step (A). Such mature adipocytes can be obtained by, for example, centrifuging the culture suspension of step (A) and collecting the cells which float in the upper part of the supernatant. This is because mature adipocytes comprise a large amount of fats, thus have a light specific gravity and float in the upper part of supernatant when centrifuged.

In the above step (B), the method of inducing differentiation of the mature adipocytes (easy-to-dedifferentiate mature adipocytes) obtained in step (A) to obtain a mesenchymal cell line derived from a vertebrate animal adipose tissue is not particularly limited as long as a method can induce dedifferentiation of the mature adipocytes to obtain a mesenchymal cell line derived from a vertebrate animal adipose tissue, but examples preferably include a method of so-called ceiling culture of the mature adipocytes. The ceiling culture is a method for culturing cells by allowing the cells to adhere or float (preferably allowed to adhere) to the inner upper surface (ceiling surface) of a culture vessel (preferably a culture flask) filled up with medium, and this method for culturing cells utilizes the property of mature adipocytes which comprise a large amount of fats, thus have a light specific gravity and float in the medium.

Examples of the medium when carrying out the dedifferentiation inducing culture of a mature adipocyte include basal medium for mesenchymal cell culture comprising the extracellular matrix, and examples of the extracellular matrix include one or more components selected from collagen, fibronectin, proteoglycan, laminin, and serum (FBS), and BD Matrigel (registered trademark) (manufactured by BD Biosciences) comprising such a component can also be used. Serum such as FBS in medium when carrying out the dedifferentiation inducing culture of a mature adipocyte may be used only as an adhesion factor for allowing a mature adipocyte to adhere to the ceiling surface of a culture vessel or may not be used only as the adhesion factor for that purpose. The medium when carrying out the dedifferentiation inducing culture of a mature adipocyte may not comprise serum such as FBS, but in light of producing a mesenchymal cell line derived from a vertebrate animal adipose tissue more efficiently, it is preferable to comprise serum such as FBS with the extracellular matrix other than serum or without the extracellular matrix other than serum. The serum concentration, in the case where the medium comprises serum such as FBS, is not particularly limited as long as a mesenchymal cell line derived from a vertebrate animal adipose tissue is obtained but examples include a range from 3 to 30 wt%, preferably include a range from 7 to 25 wt%, more preferably include a range from 7 to 13 wt%.

In the above step (B), when the conditions, other than the ceiling culture conditions, for culturing a mature adipocyte in the basal medium for mesenchymal cell culture comprising the extracellular matrix are described, examples of the culture temperature typically include a range from 12 to 45°C, preferably a range from 15 to 37°C, and examples of the culture period include a range from 2 to 28 days, preferably a range from 4 to 21 days, more preferably a range from 5 to 14 days, further preferably a range from 6 to 10 days, more preferably for 7 days, in light of balancing the production of a mesenchymal cell line derived from a vertebrate animal adipose tissue between more efficiently and in a shorter period of time. In the culture, a mature adipocyte may or may not be subcultured.

A mesenchymal cell line derived from a vertebrate animal adipose tissue may or may not be isolated from the medium after the ceiling culture in the above step (B), but it is preferable to isolate. When the ceiling culture is continued, mature adipocytes gradually decrease while the established mesenchymal cell line derived from an adipose tissue actively proliferates and a cell population comprising a large amount of mesenchymal cell lines derived from an adipose tissue can be obtained. For example, when the ceiling culture is continued for about 14 days, a cell population containing an extremely large amount of mesenchymal cell lines derived from an adipose tissue can be obtained.

The ceiling culture in the above step (B) include, for the sake of convenience, the continuation of culture in which after the mature adipocyte (easy-to-dedifferentiate mature adipocyte) obtained in step (A) is adhered to the ceiling surface of a culture vessel, the culture vessel is arranged in such a way that the adhesion surface turns to the bottom side thereof, however, the culture may be continued while the mature adipocyte (easy-to-dedifferentiate mature adipocyte) obtained in step (A) are adhered to the ceiling surface of a culture vessel to obtain a mesenchymal cell derived from an adipose tissue without carrying out the culture in which the culture vessel is arranged in such a way that the adhesion surface turns to the bottom side of the medium.

### (Mesenchymal stem cell line derived from adipose tissue according to present invention)

The mesenchymal stem cell line derived from an adipose tissue (ASCL) according to the present invention is not particularly limited as long as it is a mesenchymal stem cell line derived from a vertebrate animal adipose tissue having been produced by the method for producing a cell line according to the present invention. ASCL owned by AdipoSeeds K.K. is particularly preferable.

The ASCL according to the present invention does not spontaneously differentiate when cultured in the typical basal medium for mesenchymal cell culture which does not have a differentiation inducing action, is suitable for long-term subculture, and maintains proliferation potency and differentiation potency into mesodermal cells (one or more cells selected from the group consisting of a megakaryocyte/platelet, an osteoblast, a cartilage, and an adipocyte) even after long-term subculture. For example, The ASCL according to the present invention produced from a human subcutaneous adipose tissue has been observed to maintain proliferation potency even in the 20th generation and have a doubling time of 23 hours.

The ASCL according to the present invention preferably expresses one or more (preferably 3 or more, more preferably 5 or more, further preferably 7 or more, more preferably 8 or 9, most preferably 9) surface markers selected from the following surface marker group of mesenchymal cells, and does not express one or more (preferably 3 or more, more preferably 5 or more, further preferably 7 or more, more preferably 8 or 9, most preferably 9) surface markers selected from the following surface marker group of blood cells.
Surface marker group of mesenchymal cells: CD13, CD29, CD44, CD71, CD73, CD90, CD105, CD166, HLA-ABC;
Surface marker group of blood cells: CD11b, CD14, CD19, CD34, CD41, CD42b, CD45, CD56, HLA-DR;

International Society for Cellular Therapy sets conditions to define the mesenchymal stem cell as (A) to be an adherent cell, (B) to be capable of differentiating into bones, cartilages, and fats, and (C) to express surface markers of mesenchymal cells and not express surface markers of blood cells. Of the ASCLs according to the present invention, the cell lines of a preferable embodiment meet the conditions (A), (B), and (C).

The ASCL according to the present invention can be proliferated by culture in a mesenchymal stem cell proliferation medium. A commercially available product can be used as the mesenchymal stem cell proliferation medium.

The ASCL according to the present invention is preferably ASCL stimulated with calcium in light of obtaining a better therapeutic effect on hepatic disorders. Examples of a method for the calcium stimulation include a method of contacting (preferably, dipping) ASCL with an isotonic solution containing a compound containing calcium (for example, one or more compounds selected from the group consisting of calcium chloride, calcium citrate, calcium carbonate, calcium phosphate, calcium sulfate, calcium lactate, and calcium hydroxide). The contact time is not particularly limited, but is, for example, 5 minutes to 1 hour, preferably 10 to 20 minutes. The calcium concentration in the above isotonic solution is, for example, 1 to 100 mM, preferably 5 to 50 mM, more preferably 5 to 20 mM. The method of a particularly preferable embodiment involves stimulating ASCL by contact (preferably, dipping) with an isotonic solution containing 10 mM CaCl₂ for 15 minutes.

### (Application of ASCL according to present invention)

The mesenchymal stem cell line derived from an adipose tissue (ASCL) according to the present invention can be used in the treatment of hepatic disorders. The "treatment" as used herein includes not only improvement in symptoms of the hepatic disorders but suppression and/or delay of worsening of the symptoms, for example.

The "hepatic disorders" as used herein refer to disorders that inhibit liver functions due to some cause and make the liver no longer work normally. In the present invention, examples of the hepatic disorders to be treated include hepatic dysfunction after liver resection surgery or liver transplantation. Among others, for example, hepatic ischemia-reperfusion injury is preferably to be treated. The "hepatic disorders" as used herein also include a wide range of other liver diseases as long as the therapeutic agent of the present invention exerts an effect thereon.

Examples of such a liver disease include hepatic fibrosis and inflammatory liver disease. The "hepatic fibrosis" means a disease involving excessive formation of fibrous connective tissues of the liver, which may occur in a repair or healing process, etc. of liver tissues. Examples thereof include hepatic cirrhosis. The "inflammatory liver disease" means a disease that manifests a sustained or transient inflammatory state in which symptoms can be improved by relieving the inflammation. Examples of the inflammatory liver disease include viral hepatitis and autoimmune hepatitis. The liver disease also includes an acute liver disease and a chronic liver disease.

The "acute liver disease" refers to a disease in which inflammation or the like is found in hepatic tissues either for a short period or transiently and impairment is found in hepatic functions. Examples thereof include acute viral hepatitis and a drug-induced hepatic disorder.

The "chronic liver disease" refers to a disease in which necrosis or the like is found in hepatic tissues for a long period and impairment is found in hepatic functions. Examples thereof include chronic hepatitis B, chronic hepatitis C, hepatic cirrhosis, liver cancer, alcoholic liver damage, primary biliary cirrhosis, autoimmune hepatitis, and NASH (nonalcoholic steatohepatitis).

Examples of other liver diseases described above include a liver disease involving the activation of NLRP3 inflammasome, and a liver disease involving increase in IL-1 release. Whether a certain liver disease is a liver disease involving the activation of NLRP3 inflammasome can be determined by confirming that the degree of activation of NLRP3 inflammasome is high as compared with a normal control. Whether a certain liver disease is a liver disease involving increase in IL-1 release can be determined by confirming that the degree of IL-1 release is high as compared with a normal control.

The classes of the above diseases are non-mutually exclusive, and one disease can belong to a plurality of classes. For example, hepatocellular carcinoma is one kind of inflammatory disease and can also be regarded as one kind of hepatic fibrosis.

The therapeutic agent of the present invention is not particularly limited as long as it contains the mesenchymal stem cell line derived from an adipose tissue (ASCL) according to the present invention as an active ingredient. The therapeutic agent of the present invention is capable of being prepared, stored, and administered with reference to findings about conventionally known cell preparations and in the same manner as therein. The therapeutic agent of the present invention is not particularly limited by a dosage form as long as a therapeutic effect can be obtained. The therapeutic agent of the present invention may be, for example, in an injectable or cell sheet form as well as a form in which ASCL is contained in a liver perfusate. The cell sheet refers to a sheet-shaped cell group having one or more layers obtained by culture on a cell culture substrate and separation from the cell culture substrate.

When the therapeutic agent of the present invention is an injectable agent, examples of the method for administering the therapeutic agent of the present invention include an administration method such as infusion into the liver and/or a surrounding tissue (e.g., the spleen) in a vertebrate subject (preferably a human patient), and intravenous administration. When the therapeutic agent of the present invention is a sheet agent, examples thereof include an administration method of applying it to the surface of the liver in a vertebrate subject (preferably a human patient). For administration using a liver reperfusate, examples thereof include a method of allowing ASCL to be contained in a reperfusate, and perfusing the reperfusate.

In the case of preparing the therapeutic agent of the present invention as an injectable agent, the injectable agent can be prepared by mixing the ASCL according to the present invention with a pharmaceutically acceptable carrier, for example. Examples of the carrier include physiological saline, and injectable distilled water rendered isotonic by the addition of glucose or other aids (for example, D-sorbitol, D-mannitol, and sodium chloride). The therapeutic agent of the present invention may be further supplemented with a buffer (for example, a phosphate buffer solution and a sodium acetate buffer solution), a soothing agent (e.g., benzalkonium chloride and procaine hydrochloride), a stabilizer (e.g., human serum albumin and polyethylene glycol), a preservative, an antioxidant, or the like.

In the case of preparing the therapeutic agent of the present invention in a cell sheet form, a known method for producing a cell sheet (see, for example, Domestic re-publication No. 2017/130802 of PCT international application) can be used. Examples of such a production method include a method of culturing cells on a stimulus-responsive culture substrate coated with a polymer whose molecular structure varies depending on a stimulus such as temperature, pH, or light, and changing the surface of the stimulus-responsive culture substrate by changing conditions of the stimulus such as temperature, pH, or light, thereby separating the cells in a sheet shape from the stimulus-responsive culture substrate while maintaining the adherent state of the cells, and a method of culturing cells on an arbitrary culture substrate, and physically separating the cells from the end of the culture substrate using tweezers or the like. A preferable form is a method using, as a stimulus-responsive culture substrate, a temperature-responsive culture substrate surface-coated with a polymer whose hydration force varies in a temperature range of 0 to 80°C. The method involves culturing cells on a temperature-responsive culture substrate in a temperature region where the hydration force of the polymer is weak, then culturing the cells by changing the temperature of the medium such that the hydration force of the polymer becomes strong, and separating and recovering the cells in a sheet shape. In this respect, the cells are cultured on a cell culture substrate surface-coated with a polymer whose hydration force varies in a temperature range of 0 to 80°C, in a temperature region where the hydration force of the polymer is weak. The temperature region is usually a temperature at which the cells are cultured, preferably, for example, 33°C to 40°C. The temperature-responsive polymer may be any of a homopolymer and a copolymer. Examples of such a polymer include a polymer described in Japanese unexamined Patent Application Publication No. H2-211865.

In the case of administering the therapeutic agent of the present invention contained in a reperfusate, ASCL contained in the reperfusate can be used.

A plurality of dosage forms of the therapeutic agent of the present invention may be used in combination. The therapeutic agent in two or more dosage forms selected from the group consisting of, for example, an injectable agent, a cell sheet, and a form of ASCL contained in a liver perfusate may be used in combination. In the case of using an injectable agent as well, the injectable agent may be administered to not only one site but a plurality of sites.

The therapeutic agent of the present invention may optionally further comprise, in addition to the ASCL according to the present invention, various additive components generally used and other drugs effective for treatment of the hepatic disorders.

The therapeutic target in the treatment of the hepatic disorders according to the present invention is not particularly limited as long as it is a vertebrate animal. Examples thereof include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable.

The concentration of the ASCL contained in the therapeutic agent of the present invention can be appropriately determined depending on the dose range of the ASCL, the number of medications, etc. Examples thereof include 1 × 10⁵ cells/mL to 5 × 10⁸ cells/mL and 1 × 10⁶ cells/mL to 2 × 10⁸ cells/mL.

The dose range of the ASCL is not particularly limited and may be appropriately set according to the type of the recipient vertebrate animal, the type of hepatic disorders, the properties (body weight, age, medical condition, and the presence or absence of use of other drugs, etc.) of the recipient, and the decision of a doctor in charge, etc.

When the therapeutic target in the treatment of the diseases according to the present invention is a human, the number of cells of the ASCL to be administered per dose, albeit depending on an administration method, is, for example, 1 × 10⁶ or more cells, preferably 5 × 10⁶ or more cells, more preferably 1 × 10⁷ or more cells, further preferably 2 × 10⁷ or more cells, as the lower limit, and is, for example, 2 × 10⁸ or less cells or 1 × 10⁸ or less cells as the upper limit. When the therapeutic target is a non-human vertebrate animal, the dose (the number of cells) of ASCL appropriate for the body weight (kg) of the therapeutic target vertebrate animal can be calculated as a guideline on the basis of a numeric value obtained by converting the number of ASCL listed above to a value per kg body weight with the human body weight defined as 70 kg.

The number of doses of the therapeutic agent of the present invention is not particularly limited and may be appropriately set according to the type of the hepatic disorders, the properties (body weight, age, medical condition, and the presence or absence of use of other drugs, etc.) of the recipient, and the decision of a doctor in charge, etc. The therapeutic agent of the present invention may be administered only once or may be administered twice or more, for example. In the case of administering the therapeutic agent of the present invention twice or more, the dosing interval from previous administration can be appropriately determined according to symptoms of the hepatic disorders of the target vertebrate animal, etc. Examples thereof include an interval within the range of 3 weeks to 6 weeks and an interval within the range of 6 months to 1 year.

Hereinafter, the present invention will be described in detail with reference to Examples. However, the present invention is not limited by these examples.

### Examples

### 1 Material and method

### 1.1 Animal

Male Wistar rats (7 weeks old, body weight: 200 to 250 g) were obtained from Sankyo Labo Service Corp. All animal experiments were conducted in accordance with National Institutes of Health Guide for the Care and Use of Laboratory Animals (NIH Publication No. 8023, revised 1978) . All experimental procedures were approved by the ethics committee of Keio University.

### 1.2 ASCL and ASC

ASCL and ASC were prepared in the same manner as in the document (Tozawa K, Ono-Uruga Y, Yazawa M, et al., Megakaryocytes and platelets from a novel human adipose tissue-derived mesenchymal stem cell line. Blood. Feb 14 2019; 133 (7): 633-643. doi:10.1182/blood-2018-04-842641). Briefly speaking, subcutaneous adipose tissues obtained from a Wistar rat under isoflurane (manufactured by Pfizer Japan Inc.) anesthesia were chopped and then digested with collagenase IV (manufactured by Sigma-Aldrich). Then, the digested adipose suspension was filtered through a 70 mm mesh and centrifuged to obtain stromal vascular fraction (SVF) pellets. Then, the SVF was resuspended in high-glucose DMEM (manufactured by manufactured by Nacalai Tesque, Inc.) supplemented with 20% FBS and 100 U/mL penicillin-streptomycin, and cultured in a 100 mm dish. The medium was replaced after 24 hours from inoculation, and selected adherent cells were used as ASC (mesenchymal stem cells derived from an adipose tissue). ASC includes mesenchymal stem cells, adipose progenitor cells, stromal cells (stroma cells), vascular endothelial cells, smooth muscle cells, fibroblasts, and the like.

ASCL was prepared from existing ASC by the following method.

Culture of differentiation induction into adipocytes was performed by culturing ASC in Rat Adipocyte Differentiation Medium (manufactured by Cell Applications, Inc.). The cells thus cultured are rich in mature adipocytes (easy-to-dedifferentiate mature adipocytes) induced by differentiation from a stromal vascular fraction containing mesenchymal stem cells, adipose progenitor cells, and stromal cells. The cells thus cultured were detached from a culture dish using trypsin. Trypsin and DMEM medium (Dulbecco's Modified Eagle's Medium, manufactured by Life Technologies Corp.) were added to the cells, which were then applied to a centrifuge. Mature adipocytes (easy-to-dedifferentiate mature adipocytes) floating in the supernatant were recovered. The above easy-to-dedifferentiate mature adipocytes were added to a culture flask containing a sufficient amount of DMEM medium containing 20% FBS. The cells were cultured by allowing the cells to float or attach to the inner upper surface of the culture flask filled up with the medium (so-called "ceiling culture"). By the ceiling culture, a mesenchymal stem cell line derived from an adipose tissue (ASCL) derived from a rat was obtained.

1.3 Differentiation into adipocyte and osteoblast

ASCL and ASC were allowed to differentiate into osteoblasts in Osteogenic Differentiation Medium (manufactured by PromoCell GmbH). ASCL was allowed to differentiate into mature adipocytes in Adipocyte Differentiation Medium (manufactured by Cell Applications, Inc.), and the lipid accumulation of the adipocytes was visualized using Oil Red o (manufactured by Nacalai Tesque, Inc.) in accordance with the manufacturer's protocol.

### 1.4 Analysis on characteristics of ASCL and ASC, and determination of RNA sequence in ASCL and ASC

Passage-3 ASCL and ASC were collected and subjected to flow cytometry and RNA sequence analysis. In order to analyze the characteristics of ASCL and ASC in flow cytometry, CD44 and CD90 serving as markers unique to MSC and CD34 and CD45 serving as nonpositive markers for MSC were analyzed by flow cytometry. Specifically, detection was performed in BD FACS flow cytometry system using CD44-FITC (manufactured by BioLegend, Inc.), CD90-APC (manufactured by BioLegend, Inc.), CD34 (manufactured by Santa Cruz Biotechnology), and CD45-PE (manufactured by BioLegend, Inc.).

As for RNA sequences, a sequence library was constructed using total RNA obtained from each sample. This construction was performed using SureSelect Strand Specific RNA Library Preparation Kit for Illumina (manufactured by Agilent Technologies Inc.) in accordance with its protocol.

The quality of the library was evaluated using Agilent 2200 TapeStationD1000/High Sensitivity (manufactured by Agilent Technologies Inc.). The pooled library of the sample was sequenced with 51 bp single-end reads using HiSeq system (manufactured by Illumina, Inc.).

In functional concentration analysis, all differentially expressed genes (DEGs) between ASCL and ASC were mapped to terms of a gene ontology (GO) database, and significantly concentrated GO terms were searched for from among the DEGs with p < 0.05 as a threshold.

### 1.5 Isolation and coculture of rat hepatocyte

Hepatocytes were isolated as follows: a Wistar rat was anesthetized with isoflurane. An 18 G catheter was inserted to the portal vein, and collagenase IV (manufactured by Sigma Aldrich) was added thereto. Perfusion was performed with 150 mL of HBSS (Hanks' Balanced Salt Solution). The vena cava was immediately cut, and a perfusate was flowed. The digested liver was shaken by hand so that hepatocytes were dispersed. Then, the suspended hepatocytes were filtered through a 100 mm mesh filter. The separated hepatocytes were subjected to the Percoll density gradient centrifugation method (manufactured by GE Healthcare). The viability of the final hepatocytes was determined by Trypan Blue staining. The cells were inoculated at 5 × 10⁶ cells/well to a 6-well plate coated with collagen, and cultured at 37°C in a humidified atmosphere of 5% CO₂ using high-glucose DMEM supplemented with 10% FBS, 100 U/mL penicillin-streptomycin, 25 µg/mL epithelial growth factor, 2 U/mL GlutaMAX, 1 µM dexamethasone, and 1% insulin-transferrin serum.

1.6 Cell coculture and hypoxia/reoxygenation-conditioned culture

The coculture of hepatocytes and ASC or ASCL was performed after 6 hours from attachment of isolated hepatocytes. ASC or ASCL was inoculated in an amount of 1 × 10⁶ cells/well. Monoculture was performed as a control group. 24 hours after coculture, the medium was replaced with glucose-free DMEM medium (manufactured by Nacalai Tesque, Inc.), and the coculture group or the monoculture group was cultured for 3 hours in a hypoxic state in a humidified atmosphere of 1% O₂ and 5% CO₂ in a module incubator chamber. After the hypoxic state, the medium was replaced with high-glucose DMEM containing supplements, which was brought back to a usual culture environment 1 hour later. The medium was frozen and recovered. The cultured cells were collected in a pellet form or fixed in PBS containing 4% paraformaldehyde and used in further analysis.

### 1.7 Rat hepatic ischemia-reperfusion model and cellular therapy

Hepatic warm ischemia of a non-lethal segment (70% ischemia) was performed under isoflurane (manufactured by Pfizer Japan Inc.) anesthesia by the same method as that of the document (Fujii T, Obara H, Matsubara K, et al., Oral administration of cilostazol improves survival rate after rat liver ischemia-reperfusion injury. J Surg Res. Jun 1 2017; 213: 207-214. doi:10.1016/j.jss.2017.02.020). More specifically, the bile duct, the portal vein, and the hepatic artery of the median and left liver lobes were clamped using an aneurism clip (Sugita clip; manufactured by MIZUHO Corp.). After ischemia for 60 minutes, the clip was removed, and blood flow was reperfused. ASC or ASCL cellular therapy was performed by infusing ASC or ASCL in an amount of 1 × 10⁶ cells/animal into the spleen 24 hours before hepatic ischemia-reperfusion induction. For a control group, physiological saline was infused into the spleen. At 3 hours and 24 hours after reperfusion, rats were slaughtered (n = 5 for each group), and serum and hepatic tissue samples were collected and further analyzed.

### 1.8 Hepatic function examination

Serum alanine aminotransferase (ALT) and serum aspartate aminotransferase (AST) were analyzed by SRL, Inc.

### 1.9 Implementation of Western blotting analysis and enzyme-linked immunosorbent assay (ELISA)

In order to analyze the expression of NLRP-3 (manufactured by ABclonal Inc.), caspase-1 (manufactured by Sigma-Aldrich), IL-1β (manufactured by Abcam plc), IL-18 (manufactured by R&D Systems, Inc.), IL-1RA (manufactured by Abcam plc), and β-actin (manufactured by Cell Signaling Technology, Inc.) in hepatic tissues, Western blotting was performed in accordance with the manufacturers' protocols. The concentrations of tissue growth factor -β (TGF-β) (manufactured by R&D Systems, Inc.), IL-6 (manufactured by R&D Systems, Inc.), IL-10 (manufactured by Thermo Fisher Scientific Inc.) and IL-18 (manufactured by Thermo Fisher Scientific Inc.) in serum were detected using ELISA kits in accordance with the manufacturers' protocols.

1.10 Cell image and histological study

Cultured cells fixed in paraformaldehyde were subjected to the staining of DNA fragmentation with TUNEL using DeadEnd fluorometric TUNEL system (manufactured by Promega Corp.) and the staining of albumin using Anti-Albumin Antibody (manufactured by Abcam plc). Hepatocytes were discriminated from ASC or ASCL cocultured therewith on the basis of the cells stained with albumin.

Reactive oxygen was detected from the cell cultures using ROS assay kit (manufactured by Dojindo Laboratories) in accordance with the manufacturer's protocol.

Liver tissues of each group were fixed at 4°C using a phosphate buffer solution containing 4% paraformaldehyde. Sections with a thickness of 4 um were prepared, stained with hematoxylin-eosin, and histologically examined. TUNEL was measured using DeadEnd fluorometric TUNEL system (manufactured by Promega Corp.).

### 1.11 Quantitative real-time polymerase chain reaction (qRT-PCR)

Total RNA was extracted from cocultured cells and liver tissues using QIAGEN RNeasy Plus Mini Kit (manufactured by Dusseldorf). In order to quantify expression levels of TGF-β1 (tissue growth factor-β1), IL-10 (interleukin-10), and IL-1Ra (interleukin-1 receptor antagonist), the total RNA was reverse-transcribed into cDNA using BIO-RAD iScript cDNA Synthesis Kit (manufactured by Hercules). qRT-PCR was performed using ViiA 7 realtime PCR system (manufactured by Thermo Fisher Scientific Inc.). The primers used are shown in Table 1.

**[Table 1]**

| Primer | Forward | Reverse |
|---|---|---|
| TGF-β1 | SEQ ID NO: 1; 5'-CGTTACCTTGGTAACCGGCT-3' | SEQ ID NO: 2; 5'-AGCCCTGTATTCCGTCTCCT-3' |
| IL-10 | SEQ ID NO: 3; 5'-GAAGGACCAGCTGGACAACA-3' | SEQ ID NO: 4; 5'-TCAGCTTCTCTCCCA-3' |
| IL-1Ra | SEQ ID NO: 5; 5'-CGCTTTACCTTCATCCGCTC-3' | SEQ ID NO: 6; 5'-GGGCTCTTTTGGTGTGTTGG-3' |
| IL-6 | SEQ ID NO: 7; 5'-AGAGACTTCCAGCCAGTTGC-3' | SEQ ID NO: 8; 5'-TCTGACAGTGCATCATCGCT-3' |
| GAPDH | SEQ ID NO: 9; 5'-GAAGGTCGGTGTGAACGGAT-3' | SEQ ID NO: 10; 5'-ACCAGCTTCCCATTCTCAGC-3' |

### 1.12 Evaluation of viability and LDH (lactate dehydrogenase) after H/R lesion

The viability of cultured cells was evaluated using cell counting kit-8 (manufactured by Dojindo Laboratories), and LDH in a conditioned solution was measured using LDH assay kit (manufactured by Abcam plc) in accordance with the manufacturer's protocol.

### 1.13 Data analysis

Numeric values were indicated by mean ± standard deviation (SD). The statistical significance of difference between groups was tested by the Student's test.

### 2 Results

### 2.1 Characterization of rat ASCL and ASC

Rat-derived passage-3 ASCL and ASC were analyzed for the expression of cell surface molecules by flow cytometry. Both ASCL and ASC were positive to CD44 and CD90 serving as markers unique to MSC, and were negative to CD34 and CD45 serving as non-positive markers for MSC (Figure 1b).

As a result of examining the differentiation potency of ASCL and ASC, both ASCL and ASC exhibited positivity in both Oil Red o staining and AKP staining, demonstrating that both ASCL and ASC have differentiation potency into adipocytes or osteoblasts (Figure 1a).

In order to examine overall biological functions brought about by ASCL and ASC, gene ontology (GO) was carried out. Gene ontology (GO) analysis was conducted to extract top 30 representative genes whose expression was increased in ASCL. ASCL compared with ASC was found to exert abundant functions against hypoxia and inflammatory response. ASCL was confirmed to exhibit sensitive response, particularly, to interleukin-1, which is an important inflammation-promoting cytokine in ischemia-reperfusion (Figure 2b).

### 2.2 Suppression of hypoxia/reoxygenation damage (HRD) in hepatocyte by ASCL/ASC

ASCL and ASC were evaluated for a cytoprotective effect on hepatocytes *in vitro.* Each of rat ASCL and ASC was cocultured with rat hepatocytes, and hypoxic injury was induced for 3 hours in the coculture groups and a control group (monoculture of hepatocytes), which were then brought back to usual culture conditions for promoting a reperfusion process, and incubated for 1 hour. After being inoculated to a 6-well plate, all the cells were uniformly distributed (Figure 3a). The liver cells cocultured with ASC or ASCL were surrounded by stem cells in a lobed population, whereas the liver cells of the monoculture group were uniformly scattered in the plate (Figure 3a).

The hepatocytes of the control group were separated in a large amount from the bottom of the plate after hypoxia/reoxygenation damage, whereas the adhesion of the hepatocytes was relatively maintained in the coculture group with ASC or ASCL (Figure 3b). The cells of each group after hypoxia/reoxygenation damage were analyzed for the state of apoptosis by TUNEL cell staining (Figure 3c). As a result, the apoptosis of stem cells was suppressed in both the ASCL group (p < 0.001) and the ASC group (p < 0.001) compared with the control group (Figure 3d). As for post-damage cell viability measured by CCK-8, the viability of the ASCL group and the ASC group was also confirmed to be higher (p < 0.01) than that of the control group. As a result of measuring a LDH (lactate dehydrogenase) concentration in the medium of each group, elevation in the LDH concentration in the medium was observed after HRD in the control group, whereas the LDH concentration was significantly decreased in the ASCL group (p < 0.0049) and the ASC group (p < 0.001) compared with the control group (Figure 3e).

### 2.3 Suppression of reactive oxygen species damage in hepatocyte by ASCL/ASC

The expression of reactive oxygen species (ROS) in the ASCL group, the ASC group, and the control group after HRD was detected by cell imaging intensity (Figure 4a). The results show that the expression level of reactive oxygen was significantly decreased in the ASCL group (p < 0.001) and the ASC group (p < 0.001) compared with the control group (Figure 4b).

### 2.4 Increase in expression of cytoprotective cytokine in hypoxic environment by ASCL/ASC

The mRNA gene expression of cytoprotective cytokines (TGF-β, IL-10, and IL-1Ra) in the ASCL group, the ASC group, and the control group after HRD was measured by qRT-PCR. As a result, the expression of TGF-β was increased in the ASCL group (p = 0.0012) and the ASC group (p = 0.0014) (Figure 4c), whereas the expression of IL-10 and IL-1Ra was increased only in the ASCL group (p = 0.0497 for IL-10, p = 0.0087 for IL-1Ra). These results indicated that ASCL compared with ASC has a significantly excellent cytoprotective effect.

### 2.5 Suppression of hepatic ischemia-reperfusion injury by ASCL

Rats with ischemia-reperfusion of the liver induced after ASCL or ASC cellular therapy were compared with model rats with ischemia-reperfusion of the liver that underwent prior administration of physiological saline. As a result, the suppression of elevation of an AST concentration and an ALT concentration in serum obtained 3 hours after reperfusion was observed in the ASCL group (p = 0.0022 for AST, p = 0.0066 for ALT) (Figure 5a). On the other hand, no significant difference in hepatic function was observed between the serum of the ASC group and the serum of the control group (p = 0.3021 for AST, p = 0.2640 for ALT) (Figure 5a). These results also indicated that ASCL compared with ASC has a significantly excellent cytoprotective effect.

All the groups underwent a recovery period because the serum AST and ALT levels were decreased 24 hours after reperfusion (Figure 5a). The ASCL group exhibited the most rapid recovery trend (AST: p = 0.1295, ALT: p = 0.2517) vs. the control group (AST: p = 0.0130, ALT: p = 0.0236) (Figure 5a).

On the other hand, the hepatic enzymes of the ASC group (AST: p = 0.0059, ALT: p = 0.0039) compared with a sham group exhibited a high rate of maintenance, and significantly high numeric values were observed as compared with the sham group (Figure 5a).

### 2.6 Suppression of hepatic tissue destruction and apoptosis by ASCL

In histological study, ASCL and ASC were evaluated for a therapeutic effect by H.E. staining (Figure 5b) and TUNEL fluorescent staining (Figure 5c) of liver tissues that underwent ischemia-reperfusion injury. Partial damage on the surrounding site of hepatic lobule was observed in the control group 3 hours after reperfusion, and structural destruction of hepatic lobule and cell necrosis, apoptosis, and vacuolization were observed overall in the control group and the ASC group stained with H.E. 24 hours after reperfusion (Figure 5b). On the other hand, the lesion of the ASCL group was confirmed to a narrow range of hepatic lobule (Figure 5b). As a result of quantifying hepatic disorders of each group in accordance with Suzuki score, the ASCL group and the ASC group exhibited a significantly low score (p < 0.001) with respect to the control group 3 hours after reperfusion, and the ASCL group and the ASC group exhibited a significantly low score (p <0.001 in the ASCL group, p = 0.0337 in the ASC group) with respect to the control group 24 hours after reperfusion (Figure 5d). Further, the ASCL group also exhibited a significantly low score (p = 0.0175) with respect to the ASC group 24 hours after reperfusion (Figure 5d). These results also indicated that ASCL compared with ASC has a significantly excellent cytoprotective effect.

Similar results were obtained in the TUNEL test. More specifically, TUNEL-positive stain was detected in a large amount in the control group at 3 hours and 24 hours after reperfusion, whereas the expression of TUNEL was inhibited in the ASCL group (p < 0.001) and the ASC group (p < 0.001)

### (Figure 5e).

### 2.7 Influence of ASCL/ASC on cytokine expression at time of hepatic ischemia-reperfusion injury

The expression of cytokines in hepatic tissues and serum was measured by qRT-PCR and ELISA, respectively. In the ASCL group, significant elevation of the mRNA expression of IL-1Ra was observed with respect to the control group and the ASC group (p = 0.0028 vs control, p = 0.0090 vs ASC) (Figure 6a). In the ASCL group and the ASC group, significant decrease in the mRNA expression of the cytokine IL-6 involved in the promotion of inflammation and liver fibrosis was observed with respect to the control group (p < 0.001) (Figure 6a). In the ASCL group, significant increase in the mRNA expression of TGF-β and IL-10 was observed with respect to the control group (p = 0.0012 for TGF-β, p < 0.001 for IL-10) (Figure 6a).

In the serum ELISA, the control group had significantly high levels of IL-18 (p < 0.001 vs ASCL, p = 0.0171 vs ASC) and IL-6 (p < 0.001) and a significantly low level of TGF-β (p = 0.0185 vs ASCL, p = 0.0158 vs ASC) (Figure 6b). On the other hand, the level of IL-10 was significantly high in the ASCL group compared with the control group (p < 0.001) (Figure 6b).

### 2.8 Inhibition of NLRP3 inflammasome and activation of IL-1RA expression by ASCL

In order to further reveal a therapeutic effect on hepatic ischemia-reperfusion injury by the pretreatment of ASCL, analysis was pursued on a mechanism underlying the action of ASCL and ASC suppressing the pathological condition of hepatic ischemia-reperfusion injury. Specifically, in order to analyze the behavior of NLRP3 (NOD-like receptor family pyrin domain-containing 3) inflammasome in hepatic tissues with hepatic ischemia-reperfusion injury, the protein expression of NLRP3, caspase-1 (casp-1), IL-1β, IL-18, and IL-1Ra in the above hepatic tissues was examined by Western blot. IL-1β and IL-18 are finally activated products of NLRP3 inflammasome.

As a result, the expression of NLRP3 and fragmented caspase-1 serving as its intermediate product was elevated in the control group and tended to be decreased in the ASCL group (p < 0.001 for NLRP3, p = 0.0324 for casp-1) and the ASC group (p < 0.001 for NLRP3, p = 0.0036 for casp-1) (Figures 7a and 7c). Relative expression of IL-1Ra was elevated in the ASCL group (p < 0.001) and by contrast, significantly reduced in the ASC group (p < 0.0274) (Figures 7b and 7c). The expression of pro-IL-1β and IL-1β was suppressed in the ASCL group (p < 0.001 for pro-IL-1β, p = 0.0026 for IL-1β) and the ASC group (p < 0.001 for pro-IL-1β, p < 0.001 for IL-1β). Relative expression of pro-IL-18 was reduced in the ASCL group (p = 0.0071) and the ASC group (p < 0.001).

### 2.8 Summary of results

In the experiments of the present Examples, ASCL and ASC were evaluated for a therapeutic effect on liver ischemia-reperfusion injury by both *in vitro* and *in vivo* experiments. In the in vitro experiments of liver cell coculture and hypoxia/reoxygenation damage, the ASCL group and the ASC group had higher viability of liver cells than that in the control group, and alleviated ROS damage. The ASCL group exhibited elevated gene expression of TGF-β and IL-10. In the *in vivo* experiment of rat liver ischemia-reperfusion injury, serum biochemistry and liver histological examination after reperfusion were studied. The results show that serum AST and ALT of the ASCL group were significantly improved as compared with the control group. On the other hand, no statistically significant difference was observed in the ASC group. Genetic examination indicated that the ASCL group and the ASC group manifested an effect of the same tendency to elevate the expression level of TGF-β and to suppress the expression levels of IL-1β, IL-6, and IL-18, whereas the expression of IL-10 was significantly elevated in the ASCL group.

These results indicated that in the treatment of rat liver ischemia-reperfusion injury, the ASCL group compared with the ASC group has higher stability and has a better therapeutic effect such as a cytoprotective effect. The results of Western blot analysis further revealed that ASCL suppresses NLRP3 inflammasome and elevates the expression of IL-1Ra at the gene and protein levels. The NLRP3 inflammasome is recognized as an important mechanism underlying inflammation in each liver disease. Upon ischemia-reperfusion of the liver, pathogen-related molecular patterns (PAMPs) and danger-associated molecular patterns (DAMPs) are released from hepatocytes, sinusoidal endothelial cells, and Kupffer cells so that NLRP3 inflammasome is induced while downstream caspase-1 is fragmented and activated. Finally, cell necrosis called pyroptosis was induced by the activity and release of IL-1β and IL-18. Further, IL-1β binds to IL-1 receptor to expand inflammatory signals, resulting in a situation in which cell apoptosis and pyroptosis are deteriorated. IL-1Ra was confirmed to bind to IL-1 receptor and be able to suppress IL-1, IL-6, and TNF-α (tumor necrosis factor-a) released to downstream signals of the receptor. The present experiments indicated that ASCL suppresses the activation of NLRP3 inflammasome and the release of IL-1Ra, and exerts an excellent therapeutic effect on hepatic disorders by exerting the action of protecting damaged hepatic tissues from both pyroptosis and apoptosis.

### Industrial Applicability

The present invention can provide, for example, a therapeutic agent for hepatic disorders using a specific mesenchymal stem cell line derived from an adipose tissue (ASCL), the therapeutic agent being capable of being preserved and stably supplied and having a better therapeutic effect on hepatic disorders.

## Claims

1. A therapeutic agent for hepatic disorders, comprising a mesenchymal stem cell line derived from an adipose tissue as an active ingredient, the mesenchymal stem cell line having been produced by a production method comprising the following steps (A) and (B):
(A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
(B) a step of inducing dedifferentiation of the mature adipocyte obtained in step (A) to obtain a mesenchymal cell line derived from the vertebrate animal adipose tissue.

2. The therapeutic agent for hepatic disorders according to claim 1, wherein the step of inducing differentiation of one or more cells into a mature adipocyte in step (A) is a step of culturing the one or more cells in basal medium for mesenchymal cell culture comprising one or more adipose cell differentiation inducing substances selected from the group consisting of dexamethasone, isobutylmethylxanthine, insulin, and serum.

3. The therapeutic agent for hepatic disorders according to claim 1, wherein the dedifferentiation induction of the mature adipocyte in step (B) is performing ceiling culture of the mature adipocyte.

4. The therapeutic agent for hepatic disorders according to claim 1, wherein the one or more cells are cells obtained by removing the mature adipocyte from a cell population obtained by treating the vertebrate animal adipose tissue with an enzyme capable of dispersing the vertebrate animal adipose tissue cells.

5. The therapeutic agent for hepatic disorders according to claim 4, wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain.

6. The therapeutic agent for hepatic disorders according to claim 1, wherein the mesenchymal stem cell line derived from an adipose tissue expresses one or more surface markers selected from the following surface marker group of mesenchymal cells, and does not express one or more surface markers selected from the following surface marker group of blood cells:
surface marker group of mesenchymal cells: CD13, CD29, CD44, CD71, CD73, CD90, CD105, CD166, HLA-ABC;
surface marker group of blood cells: CD11b, CD14, CD19, CD34, CD41, CD42b, CD45, CD56, HLA-DR.

7. The therapeutic agent for hepatic disorders according to any one of claims 1 to 6, wherein the hepatic disorders are hepatic ischemia-reperfusion injury.

8. The therapeutic agent for hepatic disorders according to claim 1, wherein the vertebrate animal is a human.
